# EUROPEAN PATENT SPECIFICATION

(11) **EP 3 134 011 B1**
(45) Date of publication and mention of the grant of the patent: **09.09.2020**
(21) Application number: 15719280.8
(22) Date of filing: 27.04.2015
(51) Int. Cl.: A61B 17/15, A61B 17/17, A61F 2/30

(54) **COMBINED BONE CUTTING GUIDE AND SPREADER DEVICE**
KOMBINATION AUS KNOCHENSÄGENFÜHRUNG UND SPREIZWERKZEUG
COMBINATION GUIDE DE DÉCOUPE POUR OS ET OUTIL D'ECARTEMENT

(30) Priority: 25.04.2014 GB 201407342
(43) Date of publication of application: 01.03.2017
(73) Proprietor: Fusion Implants Limited, Liverpool, Merseyside L69 3GH (GB)
(72) Inventor: WALTON, Myles, Liverpool Merseyside CH64 1TA (GB)
(74) Representative: Bridge-Butler, Jeremy
(86) International application number: PCT/GB2015/051214
(87) International publication number: WO 2015/162437

(56) References cited:
- EP-A1- 2 036 509
- US-A1- 2005 273 114
- US-A1- 2008 015 605
- US-A1- 2008 262 500

## Description

The present invention relates to a combined bone cutting guide and spreader device, for use particularly, but not exclusively, in canine tibial tuberosity advancement surgery.

Tibial tuberosity advancement (TTA) is a known surgical technique for the treatment of cranial cruciate ligament (CCL) failure in canines. It involves cutting the tibia with a saw in order to define a tibial tuberosity fragment. This is then advanced to the desired angle using trial wedges, before a prosthetic wedge is implanted in the resulting gap. These wedges may be made from polymer or metal, and may be solid, as well as either mechanically located and/or porous in order to stimulate and foster bone ingrowth. The desired result is a patella tendon angle of 90 degrees, with the stifle in 135 degrees of extension.

The creation of the bone cut is usually performed using a saw guide, which comprises an elongate body provided with a linear proximal/distal slot for receiving and guiding a planar oscillating saw. The guide comprises a proximal pin and a distal pin, which are used to locate the guide in use. The pins are releasably retained in housings at opposite ends of the guide. The proximal pin is placed behind the straight patellar ligament, so it contacts the cartilage covered surface of the proximal tibia. The guide is then rotated about the proximal pin until it is in the desired location, and the shorter distal pin is then also inserted into the soft tissue adjacent the tibia. The width of the distal pin can be chosen to suit the animal and/or to adjust the final position of the saw guide. The final position will determine the eventual size of the tibial tuberosity fragment.

To secure the saw guide in place a drill bit is inserted through a locating opening provided in the saw guide adjacent to the proximal/distal slot. The drill bit is drilled into the bone to fix its position, and then it is left in situ. The cut is then made by passing the saw through the proximal/distal slot. Once this has been performed the drill bit and saw guide are removed.

The tibial tuberosity fragment is then advanced by inserting a series of trial wedges, each of which is wider than the previous one. This is performed until the tuberosity fragment is advanced to the desired angle. The prosthetic wedge is then implanted to complete the procedure.

A problem associated with this procedure is that is overly complex, and comprises many steps. In particular, the saw guide must be located and then secured in place, before it is utilised and then removed. There then follows the sequence of advancing the tibial tuberosity angle in stages by inserting several trial wedges. Therefore, a number of different implements are required, and it takes some time to perform the tibial tuberosity advancement.

Furthermore, the tibial tuberosity fragment is advanced not only by inserting the trial wedges into the cut, but also by exerting an advancement force using forceps applied anteriorly to the tibial tuberosity fragment. This can cause strain to the fragment, and requires considerable dexterity of the surgeon.

Yet another drawback of the known technique is that the tibial tuberosity advancement is limited to the size of the trial wedges, which typically come in a sequence of sizes which differ by 2mm or 3mm. As such, it may not possible to achieve exactly the desired angle.

US 2008/262500 to Collazo discloses a cutting guide for performing a bone osteotomy procedure. The cutting guide comprises a pair of arms which are pivotally connected, and a distractor operatively mounted between them. The cutting guide is adapted to be affixed to the bone with the arms parallel to one another and forming a cutting guide slot therebetween. The first arm and second arm are rotatable with respect to each other, and manipulation of the distractor creates a force between the arms causing rotation of one arm in relation to the other. As such, the cutting guide slot can be used to create a fragment, and then the arms rotated in order to advance it. However, the guide shown in Collazo is for use on humans, and its shape and configuration of technical features is inappropriate for use on canines.

The present invention is intended to overcome some of the above described problems.

Therefore, according to the present invention a combined bone cutting guide and spreader device comprises first and second arms and a hinge connecting first ends of said first and second arms together, in which second ends of said first and second arms each comprise a bone attachment mechanism, in which said first and second arms are moveable about said hinge between a guide position in which they are arranged parallel with one another and define a bone cutting guide slot therebetween, and such that said bone attachment mechanisms are adjacent to one another, and a spread position in which they are arranged at an angle to one another and said bone attachment mechanisms are spaced apart, characterised in that an angle positioning mechanism is mounted between said second ends of said first and second arms, in which said bone attachment mechanisms are located a first distance from said hinge and said angle positioning mechanism is located a second distance from said hinge which is greater than said first distance, and in which said angle positioning mechanism is operable to move said first and second arms about said hinge between said guide position and said spread position.

Thus, the combined bone cutting guide and spreader device of the present invention can be configured like the known saw guides described above, in order to facilitate the creation of the cut in the bone, but then it can also be used to spread the bone apart after the cut has been made. The bone attachment mechanisms facilitate the attachment of the two arms to the bone, and movement of the first and second arms about the hinge will therefore move the bone on either side of the cut apart.

This arrangement overcomes the problems associated with the known tibial tuberosity advancement technique, because the tuberosity fragment can be formed, and then advanced using one tool. This eliminates the steps of removing the saw guide, and then inserting a sequence of wedges into the cut to advance the tibial tuberosity fragment. In addition, as the first and second arms can be moved apart to any angle, the tibial tuberosity fragment can be advanced to any desired position.

It will be appreciated that the device of the present invention could be used in any surgical osteotomy technique which involved a cut to bone and then the angular advancement of a bone fragment. However, it finds particular application in canine tibial tuberosity advancement surgery. In such a procedure the device is oriented in a proximal/distal position for the bone cut and then spreading action, and other than when referring to the first and second arms, the word "first" is used herein to refer to the end or direction which is distal to the canine, and the word "second" is used herein to refer to the opposite end or direction which is proximal to the canine.

The mechanism for spreading the first and second arms apart is at the end proximal to the canine. It has been found that locating this mechanism at this end of the device can make it easier to operate during surgery.

Preferably the angle positioning mechanism can comprise a screw threaded rod, a nut threaded onto the rod and mounted to the second end of the first arm in a fixed axial position, and a retention member provided at the second end of the second arm. A tip of the screw threaded rod can be retained in the retention member in a freely axially rotatable manner. Therefore, the surgeon can rotate the rod in order to move the first arm away from the second arm during the procedure. Rotation of the rod drives the nut up the rod because it is in a fixed axial position in relation thereto. The rod can comprise a socket at its end which is adapted to be rotated by means of a tool, for example a hexagonal socket which can co-operate with a hexagonal key.

It will be appreciated that in order to continue to move the first and second arms apart, it is necessary for the nut and the retention member to remain axially aligned with the rod despite the change in angle between the first and second arms. Therefore, the nut can be formed as a shaft freely rotationally held between opposed first mounting points provided at the second end of the first arm, such that the nut can freely rotate about a radial axis thereof. Further, the retention member can also be formed as a shaft freely rotationally held between opposed second mounting points provided at the second of the second arm, such that the retention member can also freely rotate about a radial axis thereof.

In other respects the combined bone cutting guide and spreader device of the invention can comprise similar features to the known saw guides used in tibial tuberosity advancement surgery. In particular, the first arm can comprise a first housing at said first end thereof, and a second housing at said second end thereof. A first locating pin can be removably mountable in the first housing, and a second locating pin can be removably mountable in the second housing. These first and second pins can have a similar shape and purpose to the known distal and proximal pins referred to above.

Further, a locating opening can be provided at a first end of the bone cutting guide slot. Again, this can have a similar shape and purpose to the locating opening provided in the known saw guides referred to above.

The bone attachment mechanisms can be anything which would facilitate the attachment of the first and second arms to the bone of the canine in such a way as to allow for the forces applied to the device in use to be transmitted to the bone in order to advance the tibial tuberosity fragment. However, in a preferred construction the bone attachment mechanisms can each comprise an aperture in which a locating pin or wire is locatable. It has been found that the implantation of so-called A-wire or K-wire into the bone via these apertures is a suitable way to sufficiently attach the first and second arms to the bone to achieve the above described result. This kind of wire is widely used in surgical techniques of this kind, and is rigid enough to be capable of successfully transmitting the kinds of forces applied in use to the bone.

The bone cutting guide slot provided between the first and second arms in the guide position can be achieved simply by spacing the two arms apart from one another. However, in a preferred construction the first and second arms can each comprise a lip at their second ends, and these lips can abut against one another to close a second end of the bone cutting guide slot. This is advantageous because it delimits the size of the cut which can be made during the procedure, and in particular it prevents the saw from cutting into soft tissue beyond the bone. A first end of the bone cutting slot is closed by the hinge, or it can be closed by a further set of lips at the first ends of the first and second arms.

The invention can be performed in various ways, but one embodiment will now be described by way of example, and with reference to the accompanying drawings, in which:
Figure 1 is a perspective view of a combined bone cutting guide and spreader device not forming a part of the present invention;
Figure 2 is a plan view of the combined bone cutting guide and spreader device as shown in Figure 1 in a guide position;
Figure 3 is a plan view of the combined bone cutting guide and spreader device as shown in Figure 1 in a spread position;
Figure 4 is a perspective view of the combined bone cutting guide and spreader device as shown in Figure 1 in a first use configuration;
Figure 5 is a perspective view of the combined bone cutting guide and spreader device as shown in Figure 1 in a second use configuration;
Figure 6 is a plan view of the combined bone cutting guide and spreader device as shown in Figure 1 in said guide position in use;
Figure 7 is a plan view of the combined bone cutting guide and spreader device as shown in Figure 1 in said spread position in use;
Figure 8 is a perspective view of the combined bone cutting guide and spreader device as shown in Figure 1 in said spread position in use;
Figure 9 is a plan view of a canine tibia after the combined bone cutting guide and spreader device as shown in Figure 1 has been used;
Figure 10 is a perspective view of a combined bone cutting guide and spreader device according to the present invention;
Figure 11 is a plan view of the combined bone cutting guide and spreader device as shown in Figure 10 in a guide position in use; and,
Figure 12 is a plan view of the combined bone cutting guide and spreader device as shown in Figure 10 in a spread position in use.

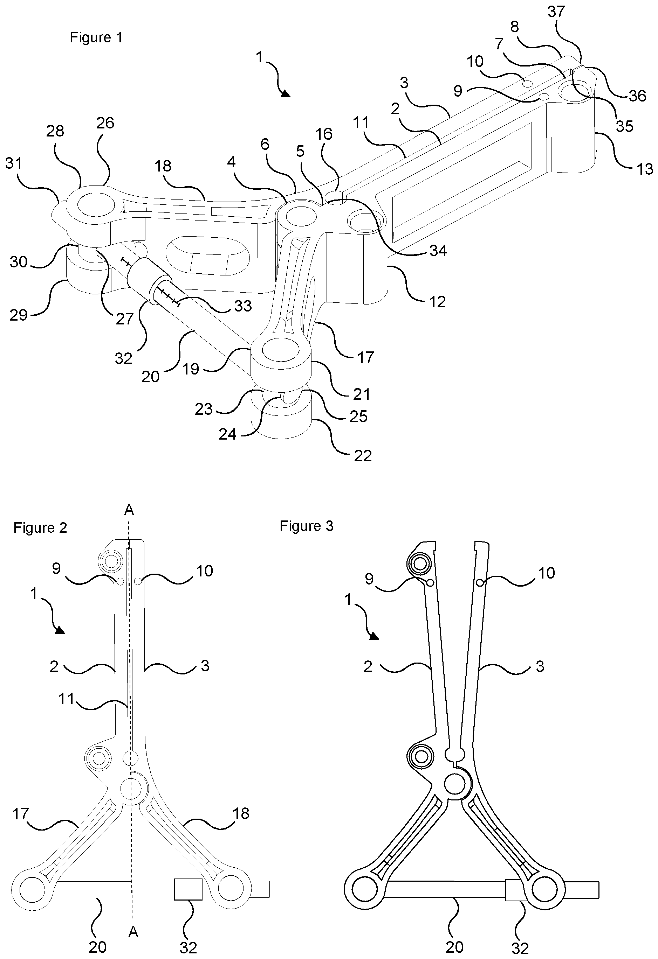

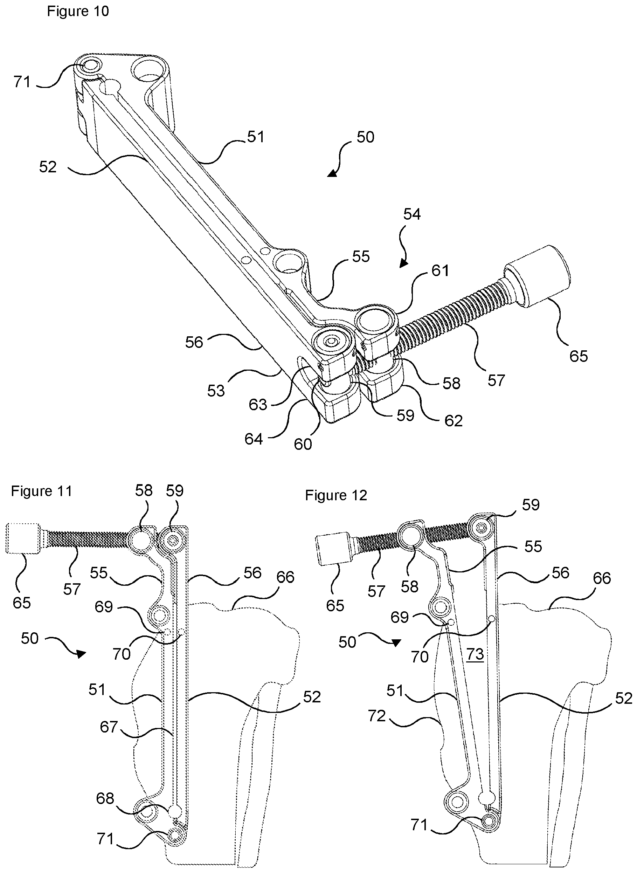

Figures 1 to 9 show a combined bone cutting guide and spreader device which does not form a part of the present invention. However, it was included in the application as filed, and a full description has been retained herein to help in the understanding of the present invention, which is a development thereof and is shown in Figures 10 to 12.

As shown in Figure 1, a combined bone cutting guide and spreader device 1 comprises first and second arms 2 and 3, and a hinge 4 connecting first ends 5 and 6 of said first and second arms 2 and 3 together. Second ends 7 and 8 of the first and second arms 2 and 3 each comprise a bone attachment mechanism, in the form of apertures 9 and 10. As explained further below, the first and second arms 2 and 3 are moveable about the hinge 4 between a guide position, as shown in Figure 2, in which they are arranged parallel with one another and define a bone cutting guide slot 11 therebetween, and such that the bone attachment mechanisms (9 and 10) are adjacent to one another, and a spread position, as shown in Figure 3, in which they are arranged at an angle to one another and said bone attachment mechanisms (9 and 10) are spaced apart.

It will be appreciated from the Figures that the two arms 2 and 3 form a saw guide which is similar in shape to known saw guides, in that it is generally rectangular, and defines an elongate planar guide slot 11 suitable for creating a proximal/distal bone cut during canine tibial tuberosity advancement surgery. It will also be appreciated that the first ends 5 and 6 of the arms 2 and 3 are the ends which are distal to the canine in use, and that the second ends 7 and 8 are proximal to the canine in use.

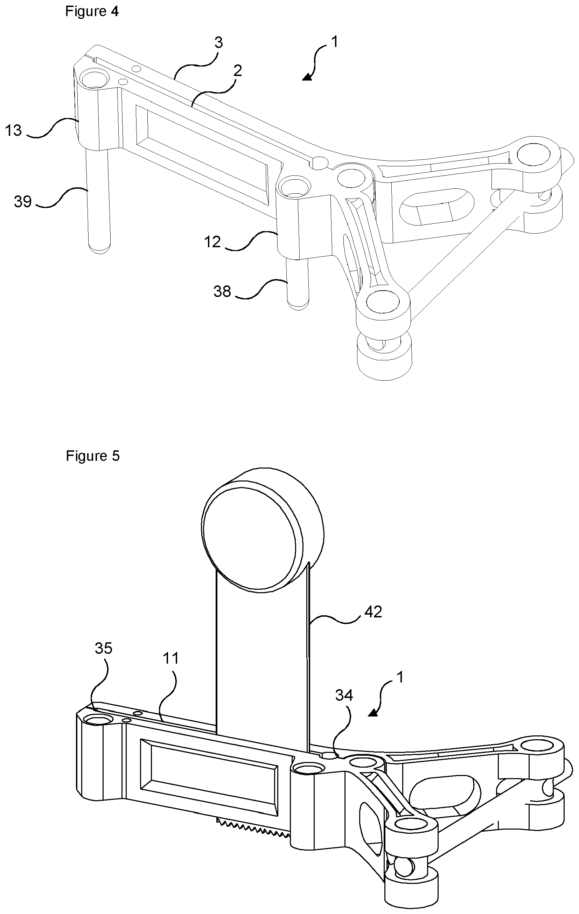

The first arm 2 comprises a first housing 12 at its first distal end 5, and a second housing 13 at its second proximal end 7. These housings 12 and 13 are for carrying locating pins so the device 1 can be positioned against the canine tibia in the known manner. Figure 4 shows a distal locating pin 38 removably mounted in the first housing 12, and a proximal locating pin 39 removably mounted in the second housing 13. These locating pins 38 and 39 have a similar shape and purpose to the known proximal and distal pins referred to above.

Further, a locating opening 16 is provided at a first end 17 of the bone cutting guide slot 11. Again, this opening 16 has a similar shape and purpose to the locating openings provided in the known saw guides referred to above.

However, unlike known saw guides, the first and second arms 2 and 3 each comprise a lever portion 17 and 18 respectively, extending from their first distal ends 5 and 6. As is clear from Figures 1 and 2, in the guide position the bone cutting guide slot 11 defines a plane, indicated by hashed line A-A in Figure 2, and the lever portions 17 and 18 are each angled away from an opposite side of the plane A-A. (Note that the device 1 is shown from the opposite side in Figures 2 and 3 to as shown in Figure 1, so the first arm 2 is on the left.)

A first end 19 of the lever portion 17 of the first arm 2 comprises a gauge rod 20. The end 19 comprises opposed axle housings 21 and 22, which support a freely axially rotatable axle 23. The axle 23 comprises a mounting aperture 24, in which an end 25 of the gauge rod 20 is mounted in a fixed position. As such, the gauge rod 20 is freely rotatable about the end 19 of the lever portion 17.

However, a first end 26 of the lever portion 18 of the second arm 3 comprises an aperture 27, through which the gauge rod 20 passes. The end 26 comprises opposed axle housings 28 and 29, which support a freely axially rotatable axle 30. This comprises the aperture 27, in which an end 31 of the gauge rod 20 is mounted in a freely sliding position. As such, the gauge rod 20 can move freely back and forth through the aperture 27 in use.

The gauge rod 20 further comprises a stop 32 mounted thereon between the lever portions 17 and 18. The stop 32 is a sleeve which is mounted on a screw thread (not shown) provided on the gauge rod 20, so it can be moved axially thereon between the ends 25 and 31 by being rotated. The stop 32 delimits the movement of the first and second arms 2 and 3 about the hinge 4 beyond the spread position, because it cannot pass through the aperture 27. This is shown in Figure 3, where the gauge rod 20 has passed through the aperture 27 until the stop 32 has come into contact with the axle 30. In this position the arms 2 and 3 are spread apart to the desired angle.

Further, the stop 32 allows for the spread position as shown in Figure 3 to be accurately pre-determined. Indicia markings 33 are provided on the gauge rod 20 which correspond to the advancement angle achieved in the spread position when the stop 32 is aligned therewith. As such, prior to use in the procedure the surgeon can rotate the stop 32 until it is aligned with the indicia 33 corresponding to the desired advancement angle.

It is important that the bone cutting guide slot 11 is closed at both ends to prevent the saw from cutting into soft tissue beyond the bone during the procedure. The first distal end 34 of the cutting slot 11 is closed by the presence of the hinge 4. In order to provide for the closure of the opposite second proximal end 35 of the cutting slot 11 the first and second arms 2 and 3 each comprise a lip 36 and 37 at their second ends 7 and 8 respectively. These lips 36 and 37 abut against one another to close the second proximal end 35 of the cutting slot 11 in the guide position.

It will be appreciated that the combined bone cutting guide and spreader device 1 can be used either way up, for operations on both the left and right rear legs of the canine. In the procedure the first arm 2 is arranged anterior to the tibia.

Therefore, in use the combined bone cutting guide and spreader device 1 is operated as follows. Firstly the surgeon must determine the angle of tibial tuberosity advancement desired for the canine being operated on. This can be achieved by examining X-rays and also be physically examining the canine before and during the procedure. Once the desired advancement angle is determined, the surgeon can set the device 1 to achieve such an angle in the spread position. He does this by rotating the stop 32 on the gauge rod 20 until it is aligned with the indicia marking 33 which corresponds with the desired advancement angle.

As in the known canine tibial tuberosity advancement technique, the device 1 is then provided with a distal pin 38 and a proximal pin 39, as shown in Figure 4. Note that the device 1 is shown the other way up in Figure 4 to as shown in Figure 1, so the first arm 2 is on the left. As such the device 1 is prepared for use on the opposite leg to as if the device 1 were oriented as shown in Figure 1. The distal pin 38 and the proximal pin 39 can be mounted in the housings 12 and 13 in either direction depending on the requirement, as the housings 12 and 13 are open at both ends.

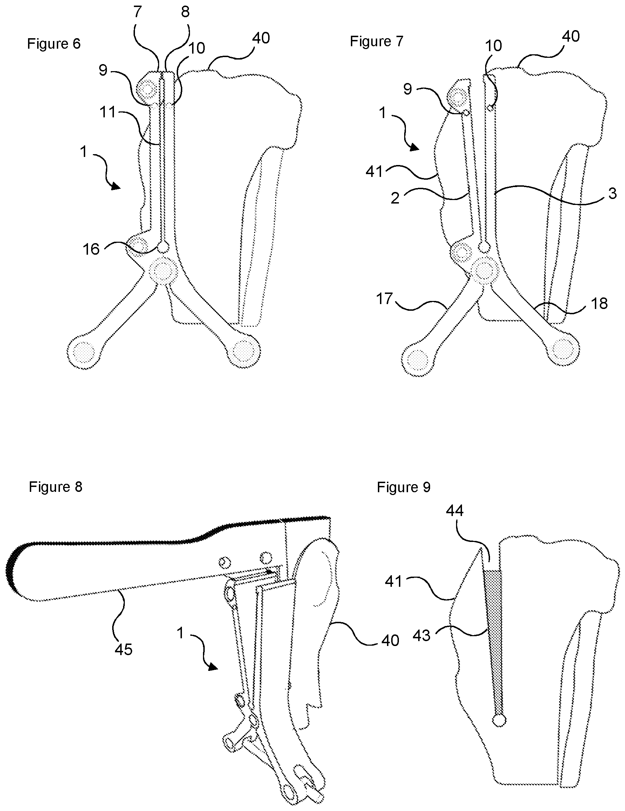

Referring to Figure 6, the device 1 is placed in the guide position and then offered up to the tibia 40, and the proximal pin 39 is placed behind the straight patellar ligament, so it contacts the cartilage covered surface of the proximal tibia 40. The device 1 is then rotated about the proximal pin 39 until the cutting slot 11 it is in the desired location, and the shorter distal pin 38 is then also inserted into the soft tissue adjacent the tibia. The width of the distal pin 38 can be chosen to suit the animal and/or to adjust the final position of the device 1. It can be replaced with an alternative during the procedure to adjust the positioning of the device 1. The final position settled upon will determine the eventual size of the tibial tuberosity fragment 41.

To secure the device 1 in place a drill bit (not shown) is inserted through the locating opening 16. The drill bit is drilled into the tibia 40 to fix the device 1 in position, and then it is left in situ.

At this point further drill holes are made in the tibia 40 through the apertures 9 and 10. Once these holes have been made K-wire (not shown) is then inserted through the apertures 9 and 10 and into the bone 40, in order to attach the second proximal ends 7 and 8 of the arms 2 and 3 thereto. This step also fixes the device 1 in the guide position.

The bone cut is then made by passing an oscillating saw 42 through the cutting slot 11, as shown in Figure 5. When the cut to the tibia 40 is made the saw 42 is prevented from cutting into the soft tissue beyond the tibia 40 by the closed ends 34 and 35 of the cutting slot 11.

Once the cut to the tibia 40 has been made the drill bit situated in the locating opening 16 is removed, and the arms 2 and 3 are then manually spread apart by applying a compression force to the lever portions 17 and 18. This force is transmitted to the tibia 40 via the arms 2 and 3, and the K-wire passing through the apertures 9 and 10 at the second proximal ends 7 and 8 thereof. As such, movement of the arms 2 and 3 towards the spread position forces the tibial tuberosity fragment 41 away from the rest of the tibia 40, as shown in Figure 7. This is done until the pre-determined spread position as shown in Figure 3 is achieved. The stop 32 comes into contact with the axle 30 and prevents any further movement apart of the arms 2 and 3. This arrangement alleviates the need for the surgeon to measure or test the advancement angle achieved during the actual procedure itself, as it can be pre-set.

It will be appreciated that the surgeon can adjust the tibial tuberosity advancement angle as this procedure is carried out. If he determines that a greater or lesser advancement angle is required then he can either set it by adjusting the stop 32 on the gauge rod 20, or he can simply position the arms 2 and 3 as desired.

It is important to note that the limiting of the spread position by the stop 32 prevents the surgeon from advancing the tibial tuberosity fragment 41 too far during the procedure, which would be a danger were such a stop mechanism not provided.

Once the desired tibial tuberosity advancement angle has been achieved a prosthetic wedge 43 is then implanted into the created gap 44 to complete the procedure. It is implanted using an implantation tool 45, as shown in Figure 8, while the device 1 is still in place on the tibia 40. The device 1 is then removed from the canine by removing the K-wire from the bone, and then withdrawing the device 1. This leaves the wedge 43 in place as shown in Figure 9, and the procedure complete.

The device 1 can be constructed from any suitable material. This can include a material which is adapted to undergo multiple sterilisation procedures, such as steel, so it can be repeatedly used. Alternatively, it can be constructed from a material which is intended to only be single-use, and discarded after use, such as a plastics material.

Figures 10 to 12 show a combined bone cutting guide and spreader device 50 according to the present invention, which is similar in construction and operation to the device 1 described above, except that the mechanism for spreading the first and second arms 51 and 52 apart is located at the opposite end of the device 50, namely the second end 53 proximal to the canine. It has been found that locating this mechanism at this end of the device 50 can make it easier to operate during surgery.

Therefore, an angle positioning mechanism, generally designated 54, is mounted between the second ends 55 and 56 of the first and second arms 51 and 52 respectively. The mechanism 54 comprises screw threaded rod 57, nut 58 threaded onto the rod 57 and mounted to the second end 55 of the first arm 51 in a fixed axial position, and a retention member 59 provided at the second end 56 of the second arm 52. The nut 58 is in a fixed axial position in relation to the rod 57 in the sense that it is held at the second end 55 of the first arm 51 in a plane which is parallel to an axis of the rod 57.

A tip 60 of the rod 57 is retained in the retention member 59 in a freely axially rotatable manner. In other words, rotation of the rod 57 is not transmitted into any axial movement of the retention member 59 in relation to the rod 57, in the way that such rotation is transmitted into an axial movement of the nut 58 in relation to the rod 57. The tip 60 of the rod 57 is retained by the retention member 59 by means of a radial flange extending therefrom (not visible).

In order to allow for the nut 58 and retention member 59 to remain axially aligned with the rod 57 when the first and second arms 51 and 52 are moved apart into a spread position, as described further below, both the nut 58 and the retention member 59 are formed as shafts which are freely rotationally held between opposed first mounting points 61 and 62 provided at the second end 55 of the first arm 51, and opposed second mounting points 63 and 64 provided at a second end 56 of the second arm 52, respectively.

The rod 57 comprises a head 65 which is provided with a hexagonal socket (not visible) which can co-operate with a hexagonal key (not shown). As such, a surgeon can located a key in the socket, and turn the head 65 in order to rotate the rod 57, and drive the nut 58 up the rod 57 towards the head 65. This moves the first and second arms 51 and 52 apart from the guide position as shown in Figure 10 to a desired spread position.

As is clear from Figure 10, in all other respects the device 50 is the same as device 1 described above, and all the identical features operate in the same way.

Therefore, the surgeon uses the device 50 in a similar way to device 1. They first determine the angle of tibial tuberosity advancement desired for the canine being operated on, however they make no adjustment to the device 50 to achieve that angle as with device 1, as it is not necessary. Distal and proximal pins (not shown) are mounted to the device 50 as required, and the device 50 is placed in the guide position and offered up to the tibia 66, as shown in Figure 11, with the proximal pin placed behind the straight patellar ligament, so it contacts the cartilage covered surface of the proximal tibia 66. The device 50 is then rotated about the proximal pin until the cutting slot 67 it is in the desired location, and the distal pin is then also inserted into the soft tissue adjacent the tibia 66. A drill bit is drilled into the tibia 66 through the locating opening 68 to fix the device 50 in position. Further drill holes are made in the tibia 66 through the apertures 69 and 70, then K-wire (not shown) is inserted therethrough into the bone 66, in order to attach the second proximal ends 55 and 56 of the arms 51 and 52 thereto. The bone is then cut with the oscillating saw through the cutting slot 67.

Once the cut to the tibia 66 has been made the drill bit situated in the locating opening 68 is removed, and the arms 51 and 52 are then spread apart by the surgeon turning the head 65 with a hexagonal key. Rotation of the head 65 rotates the rod 57, which drives the nut 58 incrementally up the rod 57. The head 65 can also be rotated manually if more convenient. As the nut 58 moves up the rod 57 the first arm 51 is moved apart from the second arm 52 about the hinge 71. This movement is transmitted to the tibia 66 via the K-wire passing through the apertures 69 and 70, and the tibial tuberosity fragment 72 is moved away from the rest of the tibia 66, as shown in Figure 12.

This is continued until the first and second arms 51 and 52 achieve the desired spread position, as shown in Figure 12. The angle between the first and second arms 51 and 52 is in line with the angle of tibial tuberosity advancement desired for the canine being operated on, as previously determined. It will be appreciated that due to the screw threaded relationship between the rod 57 and the nut 58, it is not possible for this angle to be inadvertently superseded. Instead, the angle is achieved and then maintained in a controlled way.

It will be appreciated that the surgeon can adjust the tibial tuberosity advancement angle as this procedure is carried out. If he determines that a greater or lesser advancement angle is required then he can simply rotate the head 65 in either direction to move the first arm 51 back or forth about the hinge 71.

As the angel between the first and second arms 51 and 52 increases, the nut 58 and the retention member 59 rotate on the axes of their shaft forms, between the first mounting points 61 and 62 and second mounting points 63 and 64 respectively. This prevents the nut 58 from jamming on the rod 57.

Once the desired tibial tuberosity advancement angle has been achieved a prosthetic wedge (not shown) is then implanted into the created gap 73 to complete the procedure. The device 50 is then removed from the canine by removing the K-wire from the bone, and then withdrawing the device 50.

The device 50 described above can be altered without departing from the scope of claim 1. For example, in alternative embodiments (not shown) the bone attachment mechanisms are other known arrangements including screws, nails or even adhesive.

Thus, the combined bone cutting guide and spreader device of the present invention can be configured like the known saw guides described above, in order to facilitate the creation of the cut in the bone, but then it can also be used to spread the bone apart after the cut has been made.

This arrangement overcomes the problems associated with the known tibial tuberosity advancement technique, because the tuberosity fragment can be formed, and then advanced using just the device 1. There is no step of removing a saw guide, and then inserting a sequence of wedges into the cut to advance the tibial tuberosity fragment. In addition, the tibial tuberosity advancement angle can be pre-set, and then achieved with relative ease.

## Claims

1. A combined bone cutting guide and spreader device (50) comprising first and second arms (51, 52) and a hinge (71) connecting first ends of said first and second arms (51, 52) together, in which second ends (53) of said first and second arms (51, 52) each comprise a bone attachment mechanism (69, 70), in which said first and second arms (51, 52) are moveable about said hinge (71) between a guide position in which they are arranged parallel with one another and define a bone cutting guide slot (67) therebetween, and such that said bone attachment mechanisms (69, 70) are adjacent to one another, and a spread position in which they are arranged at an angle to one another and said bone attachment mechanisms (69, 70) are spaced apart, wherein an angle positioning mechanism (54) is mounted between said second ends (55, 56) of said first and second arms (51, 52), and is operable to move said first and second arms (51, 52) about said hinge (71) between said guide position and said spread position, **characterised in that** said bone attachment mechanisms (69, 70) are located a first distance from said hinge (71) and said angle positioning mechanism (54) is located a second distance from said hinge (71) which is greater than said first distance.

2. A combined bone cutting guide and spreader device (50) as claimed in claim 1 in which said angle positioning mechanism (54) comprises a screw threaded rod (57), a nut (58) threaded onto said rod (57) and mounted to said second end (55) of said first arm (51) in a fixed axial position, and a retention member (59) provided at said second end (56) of said second arm (52), in which a tip (60) of said screw threaded rod (57) is retained in said retention member (59) in a freely axially rotatable manner.

3. A combined bone cutting guide and spreader device (50) as claimed in claim 1 in which said first arm (51) comprises a first housing at said first end thereof, and a second housing at said second end (55) thereof, and in which said combined bone cutting guide and spreader device (50) further comprises a first locating pin removably mountable in said first housing, and a second locating pin removably mountable in said second housing.

4. A combined bone cutting guide and spreader device (50) as claimed in claim 1 in which a locating opening (68) is provided at a first end of said bone cutting guide slot (67).

5. A combined bone cutting guide and spreader device (50) as claimed in claim 1 in which said bone attachment mechanisms each comprise an aperture (69, 70) in which a locating pin or wire is locatable.

6. A combined bone cutting guide and spreader device (50) as claimed in claim 1 in which said first and second arms (51, 52) each comprise a lip at said second ends (55, 56) thereof, in which in said guide position said lips abut against one another to close a first end of said bone cutting guide slot (67).

## Patentansprüche

1. Kombinierte Knochenschneidführungs- und -spreizvorrichtung (50) mit einem ersten und einem zweiten Arm (51, 52) und einem Gelenk (71), das erste Enden des ersten und des zweiten Arms (51, 52) miteinander verbindet, wobei zweite Enden (53) des ersten und des zweiten Arms (51, 52) jeweils einen Knochenbefestigungsmechanismus (69, 70) umfassen, wobei der erste und der zweite Arm (51, 52) um das Gelenk (71) zwischen einer Führungsposition, in der sie parallel zueinander angeordnet sind und einen Knochenschneidführungsschlitz (67) dazwischen definieren, und so dass die Knochenbefestigungsmechanismen (69, 70) zueinander benachbart sind, und einer gespreizten Position, in der sie in einem Winkel zueinander angeordnet sind und die Knochenbefestigungsmechanismen (69, 70) beabstandet sind, beweglich sind, wobei ein Winkelpositionierungsmechanismus (54) zwischen den zweiten Enden (55, 56) des ersten und des zweiten Arms (51, 52) montiert ist und betriebsfähig ist, um den ersten und den zweiten Arm (51, 52) um das Gelenk (71) zwischen der Führungsposition und der gespreizten Position zu bewegen, **dadurch gekennzeichnet, dass** die Knochenbefestigungsmechanismen (69, 70) in einem ersten Abstand vom Gelenk (71) angeordnet sind und der Winkelpositionierungsmechanismus (54) in einem zweiten Abstand vom Gelenk (71) angeordnet ist, der größer ist als der erste Abstand.

2. Kombinierte Knochenschneidführungs- und -spreizvorrichtung (50) nach Anspruch 1, wobei der Winkelpositionierungsmechanismus (54) eine Schraubengewindestange (57), eine Mutter (58), die auf die Stange (57) geschraubt ist und am zweiten Ende (55) des ersten Arms (51) in einer festen axialen Position montiert ist, und ein Halteelement (59), das am zweiten Ende (56) des zweiten Arms (52) vorgesehen ist, umfasst, wobei eine Spitze (60) der Schraubengewindestange (57) in dem Halteelement (59) in einer frei axial drehbaren Weise gehalten wird.

3. Kombinierte Knochenschneidführungs- und -spreizvorrichtung (50) nach Anspruch 1, wobei der erste Arm (51) ein erstes Gehäuse am ersten Ende davon und ein zweite Gehäuse am zweiten Ende (55) davon umfasst, und wobei die kombinierte Knochenschneidführungs- und -spreizvorrichtung (50) ferner einen ersten Anordnungsstift, der im ersten Gehäuse entfernbar montierbar ist, und einen zweiten Anordnungsstift, der im zweiten Gehäuse entfernbar montierbar ist, umfasst.

4. Kombinierte Knochenschneidführungs- und -spreizvorrichtung (50) nach Anspruch 1, wobei eine Anordnungsöffnung (68) an einem ersten Ende des Knochenschneidführungsschlitzes (67) vorgesehen ist.

5. Kombinierte Knochenschneidführungs- und -spreizvorrichtung (50) nach Anspruch 1, wobei die Knochenbefestigungsmechanismen jeweils einen Durchbruch (69, 70) umfassen, in dem ein Anordnungsstift oder Draht angeordnet werden kann.

6. Kombinierte Knochenschneidführungs- und -spreizvorrichtung (50) nach Anspruch 1, wobei der erste und der zweite Arm (51, 52) jeweils eine Lippe an den zweiten Enden (55, 56) davon umfassen, wobei in der Führungsposition die Lippen aneinander anliegen, um ein erstes Ende des Knochenschneidführungsschlitzes (67) zu schließen.

## Revendications

1. Dispositif d'écarteur et de guidage de coupe d'os combiné (50), comprenant des premier et deuxième bras (51, 52) et une charnière (17) reliant entre elles des premières extrémités desdits premier et deuxième bras (51, 52), des deuxièmes extrémités (53) desdits premier et deuxième bras (51, 52) comprenant chacune un mécanisme de fixation d'os (69, 70), lesdits premier et deuxième bras (51, 52) étant mobiles autour de ladite charnière (71) entre une position de guidage dans laquelle ils sont disposés de façon parallèle l'un par rapport à l'autre et ils définissent une fente de guidage de coupe d'os (67) entre ceux-ci, et de telle sorte que lesdits mécanismes de fixation d'os (69, 70) soient adjacents l'un à l'autre, et une position d'écartement dans laquelle ils sont disposés selon un certain angle l'un par rapport à l'autre et lesdits mécanismes de fixation d'os (69, 70) sont espacés l'un de l'autre, un mécanisme de positionnement d'angle (54) étant monté entre lesdites deuxièmes extrémités (55, 56) desdits premier et deuxième bras (51, 52), et pouvant fonctionner de façon à déplacer lesdits premier et deuxième bras (51, 52) autour de ladite charnière (71) entre ladite position de guidage et ladite position d'écartement, **caractérisé en ce que** lesdits mécanismes de fixation d'os (69, 70) sont situés à une première distance de ladite charnière (71) et **en ce que** ledit mécanisme de positionnement d'angle (54) est situé à une deuxième distance de ladite charnière (71), qui est supérieure à ladite première distance.

2. Dispositif d'écarteur et de guidage de coupe d'os combiné (50) selon la revendication 1, dans lequel ledit mécanisme de positionnement d'angle (54) comprend une tige filetée (57), un écrou (58) vissé sur ladite tige (57) et monté sur ladite deuxième extrémité (55) dudit premier bras (51) dans une position axiale fixe, et un élément de rétention (59) disposé à ladite deuxième extrémité (56) dudit deuxième bras (52), dans lequel une pointe (60) de ladite tige filetée (57) est retenue dans ledit élément de rétention (59) d'une façon librement rotative de façon axiale.

3. Dispositif d'écarteur et de guidage de coupe d'os combiné (50) selon la revendication 1, dans lequel ledit premier bras (51) comprend un premier boîtier à ladite première extrémité de celui-ci, et un deuxième boîtier à ladite deuxième extrémité (55) de celui-ci, et dans lequel ledit dispositif d'écarteur et de guidage de coupe d'os combiné (50) comprend de plus une première broche de positionnement pouvant être montée de façon amovible dans ledit premier boîtier, et une deuxième broche de positionnement pouvant être montée de façon amovible dans ledit deuxième boîtier.

4. Dispositif d'écarteur et de guidage de coupe d'os combiné (50) selon la revendication 1, dans lequel une ouverture de positionnement (68) est réalisée à une première extrémité de ladite fente de guidage de coupe d'os (67).

5. Dispositif d'écarteur et de guidage de coupe d'os combiné (50) selon la revendication 1, dans lequel lesdits mécanismes de fixation d'os comprennent chacun une ouverture (69, 70) dans laquelle peut être disposé une broche ou un fil de positionnement.

6. Dispositif d'écarteur et de guidage de coupe d'os combiné (50) selon la revendication 1, dans lequel lesdits premier et deuxième bras (51, 52) comprennent chacun une lèvre auxdites deuxièmes extrémités (55, 56) de ceux-ci, dans lequel, dans ladite position de guidage, lesdites lèvres butent l'une contre l'autre de façon à fermer une première extrémité de ladite fente de guidage de coupe d'os (67).
